(19) Europäisches Patentamt
European Patent Office
Office européen des brevets

(11) Veröffentlichungsnummer: **0 464 541 A2**

# (12) EUROPÄISCHE PATENTANMELDUNG

(21) Anmeldenummer: **91110332.3**

(51) Int. Cl.5: **A61C 13/20**, A61K 6/04

(22) Anmeldetag: **22.06.91**

(30) Priorität: **25.06.90 DE 4020125**

(43) Veröffentlichungstag der Anmeldung:
**08.01.92 Patentblatt 92/02**

(84) Benannte Vertragsstaaten:
**AT BE CH DE DK ES FR GB IT LI LU NL SE**

(71) Anmelder: **HERAEUS KULZER GMBH**
**Heraeusstrasse 12-14**
**W-6450 Hanau am Main(DE)**

(72) Erfinder: **Brämer, Wulf, Dr.**
**Georg-Kerschensteiner-Strasse 1**
**W-6454 Bruchköbel(DE)**
Erfinder: **Schusser, Udo, Dr.**
**Rhönstrasse 12**
**W-8755 Alzenau(DE)**
Erfinder: **Krämer, Winfried**
**Hauptstrasse 44-46**
**W-6482 Bad Orb(DE)**

(74) Vertreter: **Grimm, Ekkehard**
**Heraeus Holding GmbH Heraeusstrasse 12 - 14**
**W-6450 Hanau/Main(DE)**

(54) **Verfahren zur Herstellung von Gussobjekten aus Nichtedelmetall-Legierungen und Mittel dafür.**

(57) Aus Nichtedelmetall-Legierungen lassen sich dentale Gußobjekte mit sehr feinkörnigem und homogenem Gefüge herstellen, wenn die Modelle für die Gußobjekte aus ein Kornfeinungsmittel in homogener Verteilung enthaltendem Wachs und/oder Kunststoff angefertigt werden.

EP 0 464 541 A2

Die Erfindung betrifft ein Verfahren zur Herstellung von dentalen Gußobjekten aus Nichtedelmetall-Legierungen unter Verwendung eines Kornfeinungsmittels durch Anfertigen eines aus Wachs und/oder Kunststoff bestehenden Modells, Einbetten des Modells in eine feuerfeste Einbettmasse, Entfernen des Wachses und/oder Kunststoffes unter Bildung einer Gießform und Eingießen der geschmolzenen Nichtedelmetall-Legierungen in die Gießform und ein Mittel zur Anwendung in dem Verfahren.

Die Erfindung geht aus von dem in DE 38 21 204 A1 beschriebenen Verfahren zur Herstellung von Dentalgußskeletten, bei dem das auf dem aus feuerfester Einbettmasse bestehenden Zweitmodell aus Wachs und/oder Kunststoff angefertigte Modell des Dentalgußskeletts vor dem Einbetten in die feuerfeste Einbettmasse mindestens teilweise mit einer Kornfeinungsmittel enthaltenden, streichfähigen Impfmittelmischung beschichtet wird. Nach Entfernen des Wachses und/oder Kunststoffes bleibt die Impfmittelschicht als Oberfläche der Gußform erhalten. Während des Erstarrens der Schmelze der die Dentalgußskelette bildenden Nichtedelmetall-Legierungen dringen die in der Impfmittelschicht enthaltenen Kornfeinungsmittel in die Schmelze ein und regen dort eine verstärkte Feinkornbildung an. Dies führt in diesen Bereichen zu einer Aushärtung des Gußskelettmaterials. Die nach dem Verfahren hergestellten Dentalgußskelette weisen kleinere Kristallbereiche und bessere mechanische Eigenschaften auf als solche, die ohne die Impfmittel hergestellt werden.

Die bei dem Verfahren benutzte Impfmittelmischung liegt vorzugsweise als wässerige Suspension vor und enthält neben dem Kornfeinungsmittel noch eine anorganische Komponente und eine ausbrennbare organische Komponente als Bindemittel.

Abgesehen davon, daß sich beim Trocknen der Impfmittelmischung auf dem Modell unter Umständen Risse bilden können, besteht die Gefahr, daß sich das Modell durch das Beschichten mit der Impfmittelmischung verzieht und so die Paßgenauigkeit der Gußobjekte leidet.

Es ist daher die Aufgabe der Erfindung, ein Verfahren der eingangs charakterisierten Art zu finden, das, ohne ihre Paßgenauigkeit zu beeinträchtigen, zu Gußobjekten mit feinkörnigem Gefüge führt. Zusätzliche Verfahrensschritte, wie das Auftragen und Aushärtenlassen der Impfmittelmischung, sollen dabei vermieden werden. Außerdem soll ein Mittel zur Anwendung in dem Verfahren bereitgestellt werden.

Das die Lösung der Aufgabe darstellende Verfahren ist erfindungsgemäß dadurch gekennzeichnet, daß das Modell aus das Kornfeinungsmittel in homogener Verteilung enthaltendem Wachs und/oder Kunststoff angefertigt wird.

Bewährt hat sich das Verfahren, wenn das Kornfeinungsmittel zu 0,5-20 Gewichts-%, vorzugsweise zu 3-10 Gewichts-%, in dem Wachs und/oder Kunststoff enthalten ist, und wenn die Teilchengröße des Kornfeinungsmittels unterhalb von 100 Mikrometer, vorzugsweise im Bereich von 2 - 50 Mikrometer, liegt.

Grundsätzlich können bei dem erfindungsgemäßen Verfahren alle auch für das Verfahren nach DE 38 21 204 A1 geeigneten Kornfeinungsmittel eingesetzt werden. Bevorzugt werden jedoch Kobalt-titan-silicat, Kobalt-zirkonium-silicat, Titandioxid und Zirkoniumdioxid, wobei ersteres sich besonders bewährt hat, vor allem beim Vergießen von Dentallegierungen auf Kobalt-Chrom-Molybdän-Basis. Jedes Kornfeinungsmittel kann sowohl für sich allein als auch im Gemisch mit einem oder mehreren anderen verwendet werden.

Nach dem Entfernen des Wachses und/oder Kunststoffes befinden sich die Kornfeinungsmittel-Teilchen - anders als bei dem bekannten Verfahren - in nur lose anhaftender Form auf der Oberfläche des Gießformhohlraumes. Sie werden beim Gießen von der in die Gießform einströmenden Schmelze erfaßt, von der Schmelze aufgenommen und durch die Bewegung der Schmelze gleichmäßig darin verteilt. So kann sich beim Erstarren der Schmelze sowohl ein sehr feinkörniges als auch sehr homogenes Gefüge ausbilden. Entsprechend zeichnen sich die nach dem Verfahren hergestellten Gußobjekte durch sehr gute mechanische Eigenschaften, wie Bruchdehnung, Zugfestigkeit und Streckgrenze, aus. Ihre Paßgenauigkeit ist sehr gut.

Das Mittel zur Anwendung in dem Verfahren ist erfindungsgemäß dadurch gekennzeichnet, daß es auch Wachs oder Kunststoff und darin homogen verteiltem Kornfeinungsmittel besteht.

Bewährt hat sich das Mittel, wenn es 0,5-20 Gewichts-%, vorzugsweise 3-10 Gewichts-%, des Kornfeinungsmittels enthält und wenn die Teilchengröße des Kornfeinungsmittels unterhalb von 100 Mikrometer, vorzugsweise im Bereich von 2 - 50 Mikrometer, liegt.

Es kann jedes der auch für die Impfmittelmischung nach DE 38 21 204 A1 geeigneten Kornfeinungsmittel enthalten. Bevorzugt werden jedoch Kobalt-titan-silicat, Kobalt-zirkonium-silicat, Titandioxid und Zirkoniumdioxid, wobei ersteres sich besonders bewährt hat. Jedes Kornfeinungsmittel kann sowohl für sich allein als auch im Gemisch mit einem oder mehreren anderen in dem Wachs oder Kunststoff enthalten sein.

Für das Mittel können alle in der Dentaltechnik üblicherweise als Modellierwerkstoffe benutzten, rückstandslos verbrennenden Wachse und Kunststoffe verwendet werden. Die Herstellung des Mittels erfolgt durch Vermischen des verflüssigten Wachses oder Kunststoffes mit dem Kornfeinungsmittel. Bewährt hat sich das Mittel in Form von Platten, Drähten, Gittern und Käppchen.

Das Mittel gemäß der Erfindung schafft die Möglichkeit, nach in der Dentaltechnik herkömmlichen Verfahren Gußobjekte herzustellen, ohne daß durch die Anwendung des Kornfeinungsmittels zusätzliche Arbeitsmittel erforderlich sind und einzelne Verfahrensschritte geändert bzw. neue ergänzt werden müssen. Als überraschend im Vergleich zu der sowohl in der Zusammensetzung als auch in der Anwendung aufwendigeren Impfmittelmischung nach DE 38 21 204 A1 muß es angesehen werden, daß durch die Kombination des Kornfeinungsmittels mit einem in den herkömmlichen Verfahren benutzten Arbeitsmittel - dem Modellierwerkstoff - Gußobjekte mit feinkörnigem und homogenem Gefüge und hoher Paßgenauigkeit in gewohnter Weise hergestellt werden können.

Mit dem Verfahren und dem Mittel gemäß der Erfindung können aus Nichtedelmetall-Legierungen bestehende Zahnprothesenplatten, Kronen, Brükken, Inlays und Onlays hergestellt werden.

Zur näheren Erläuterung wird in dem folgenden Beispiel die Herstellung einer Zahnprothesenplatte aus einer Dentallegierung auf Kobalt-Chrom-Molybdän-Basis gemäß der Erfindung beschrieben.

Beispiel

Auf dem aus feuerfester Einbettmasse bestehenden Zweitmodell des Gebisses eines Patienten wird aus einer Platte aus 3 Gewichts-% Kobalt-titan-silicat mit einer Teilchengröße im Bereich von 2 - 50 Mikrometer in homogener Verteilung enthaltendem Wachs das Modell für eine Zahnprothesenplatte angefertigt. Das Modell wird durch Anbringen von Gußtrichter und Gußkanälen aus üblichem Modellierwachs vervollständigt und dann mit feuerfester Einbettmasse überbettet. Nach dem Aushärten der Einbettmasse wird das Wachs durch Erwärmen entfernt. Anschließend wird die so erhaltene Gießform auf 1030°C vorgewärmt und die Schmelze der Kobalt-Chrom-Molybdän-Dentallegierung Heraenium$^R$ CE (Heraenium: eingetragenes Warenzeichen der W.C. Heraeus GmbH) in die Gießform eingegossen. Nach dem Abkühlen wird die Zahnprothesenplatte ausgebettet und in bekannter Weise ausgearbeitet.

Die die Zahnprothesenplatte bildende Kobalt-Chrom-Molybdän-Legierung besitzt, wie die metallographische Untersuchung von daraus entnommenen Proben zeigt, ein sehr feinkörniges und homogenes Gefüge frei von Stengelkristallen.

**Patentansprüche**

1. Verfahren zur Herstellung von dentalen Gußobjekten aus Nichtedelmetall-Legierungenunter Verwendung eines Kornfeinungsmittels durch Anfertigen eines aus Wachs und/oder Kunststoff bestehenden Modells, Einbetten des Modells in eine feuerfeste Einbettmasse, Entfernen des Wachses und/oder Kunststoffes unter Bildung einer Gießform und Eingießen der geschmolzenen Nichtedelmetall-Legierungen in die Gießform, dadurch gekennzeichnet, daß das Modell aus das Kornfeinungsmittel in homogener Verteilung enthaltendem Wachs und/oder Kunststoff angefertigt wird.

2. Verfahren nach Anspruch 1, dadurch gekennzeichnet, daß das Kornfeinungsmittel zu 0,5-20 Gewichts-% in dem Wachs und/oder Kunststoff enthalten ist.

3. Verfahren nach Anspruch 2, dadurch gekennzeichnet, daß das Kornfeinungsmittel zu 3-10 Gewichts-% in dem Wachs und/oder Kunststoff enthalten ist.

4. Verfahren nach einem der Ansprüche 1 bis 3, dadurch gekennzeichnet, daß die Teilchengröße des Kornfeinungsmittels unterhalb von 100 Mikrometer liegt.

5. Verfahren nach Anspruch 4, dadurch gekennzeichnet, daß die Teilchengröße des Kornfeinungsmittels im Bereich von 2 - 50 Mikrometer liegt.

6. Verfahren nach einem der Ansprüche 1 bis 5, dadurch gekennzeichnet, daß das Kornfeinungsmittel aus Kobalt-titan-silicat, Kobalt-zirkonium-silicat, Titandioxid und/oder Zirkoniumdioxid besteht.

7. Mittel zur Anwendung in dem Verfahren nach einem der Ansprüche 1 bis 6, dadurch gekennzeichnet, daß es aus Wachs oder Kunststoff und darin homogen verteiltem Kornfeinungsmittel besteht.

8. Mittel nach Anspruch 7, dadurch gekennzeichnet, daß es 0,5-20 Gewichts-% des Kornfeinungsmittels enthält.

9. Mittel nach Anspruch 8, dadurch gekennzeichnet, daß es 3-10 Gewichts-% des Kornfeinungsmittels enthält.

10. Mittel nach einem der Ansprüche 7 bis 9, dadurch gekennzeichnet, daß die Teilchengröße des Kornfeinungsmittels unterhalb von 100 Mikrometer liegt.

11. Mittel nach Anspruch 10, dadurch gekennzeichnet, daß die Teilchengröße des Kornfeinungsmittels im Bereich von 2 - 50 Mikrometer liegt.

12. Mittel nach einem der Ansprüche 7 bis 11, dadurch gekennzeichnet, daß das Kornfeinungsmittel aus Kobalt-titan-silicat, Kobalt-zirkonium-silicat, Titandioxid und/oder Zirkoniumdioxid besteht.

13. Mittel nach einem der Ansprüche 7 bis 12, dadurch gekennzeichnet, daß es in Platten-, Draht-, Gitter- oder Käppchen-Form vorliegt.